**Europäisches Patentamt**

**European Patent Office**

**Office européen des brevets**

(19)

(11) Veröffentlichungsnummer: **0 363 646**

**A1**

(12) # EUROPÄISCHE PATENTANMELDUNG

(21) Anmeldenummer: **89116532.6**

(22) Anmeldetag: **07.09.89**

(51) Int. Cl.⁵: **C12M 1/04 , C12P 19/04**

(30) Priorität: **10.10.88 DE 3834443**

(43) Veröffentlichungstag der Anmeldung:
**18.04.90 Patentblatt 90/16**

(84) Benannte Vertragsstaaten:
**AT BE CH DE ES FR GB IT LI NL**

(71) Anmelder: **Gesellschaft für
Biotechnologische Forschung mbH (GBF)
Mascheroder Weg 1
D-3300 Braunschweig-Stöckheim(DE)**

(72) Erfinder: **Deckwer, Wolf-Dieter, Prof.-Dr.
Mascheroder Weg 1
D-3300 Braunschweig(DE)**
Erfinder: **Schumpe, Adrian, Dr.
Mascheroder Weg 1
D-3300 Braunschweig(DE)**
Erfinder: **Suh, Il-Soon
Mascheroder Weg 1
D-3300 Braunschweig(DE)**

(74) Vertreter: **Boeters, Hans Dietrich, Dr. et al
Boeters, Bauer & Partner
Thomas-Wimmer-Ring 14
D-8000 München 22(DE)**

(54) Verfahren zur fermentativen Herstellung exozellulärer Polysaccharide in Blasensäulen.

(57) Die Erfindung betrifft ein Verfahren zur fermentativen Herstellung exozellulärer Polysaccharide in Blasensäulen.

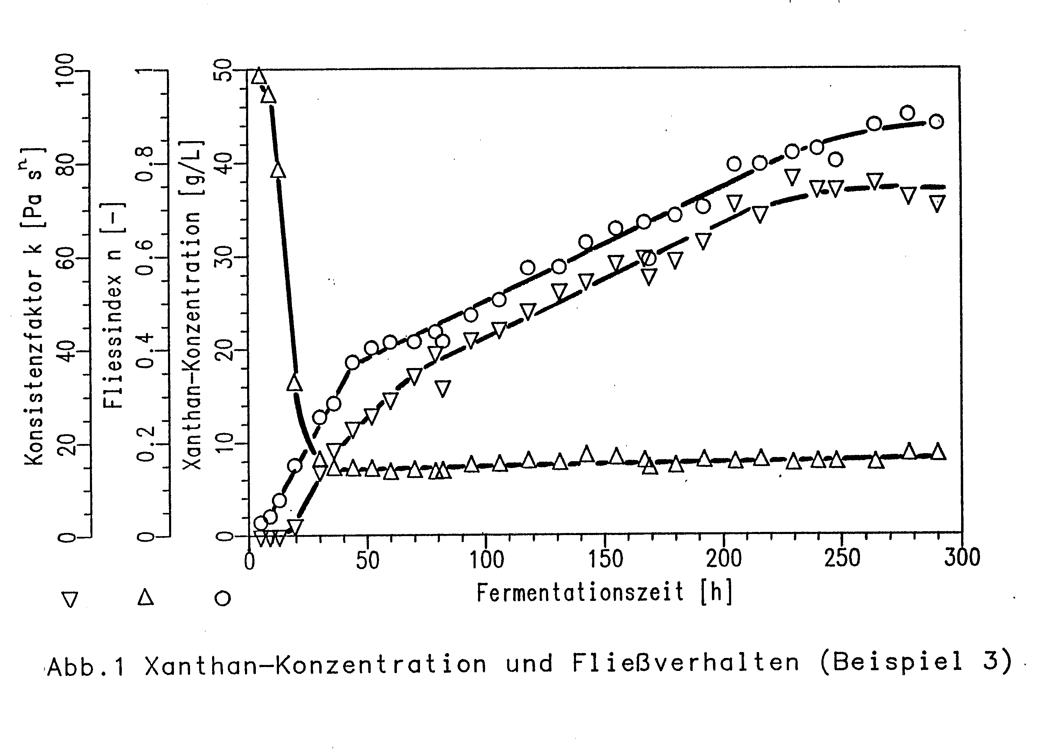

Abb.1 Xanthan-Konzentration und Fließverhalten (Beispiel 3)

**EP 0 363 646 A1**

## Verfahren zur fermentativen Herstellung exozellulärer Polysaccharide in Blasensäulen

Die fermentative Herstellung exozellulärer Polysaccharide erfolgt üblicherweise chargenweise in Rührkesselfermentern. Die Anreicherung des Produkts im Kulturmedium bewirkt einen erheblichen Anstieg der Viskosität und ein stark pseudoplastisches Fließverhalten. Beispielsweise wurde bei der Herstellung von Xanthan durch Fermentation von Xanthomonas campestris ein Anstieg des Konsistenzfaktors um mehr als den Faktor 1000 und ein Absinken des Fließindexes von annähernd 1 auf 0,16 beobachtet (Abb. 1). Der Viskositätsanstieg erschwert den Sauerstoffeintrag, den Temperaturausgleich und die Vermischung des Fermenterinhalts. Bereits in geringer Entfernung vom Rührorgan kann das Medium unbewegt sein; stagnante Zonen bilden sich insbesondere in Wandnähe und um Einbauten, wie Sonden und Strombrecher. Unter diesen Umständen können lokale Abweichungen von der optimalen Temperatur und dem optimalen pH-Wert auftreten; in den stagnanten Zonen ist auch die Versorgung aerober Mikroorganismen mit Sauerstoff nicht gewährleistet. Große Teile des Fermenterinhaltes unterliegen somit suboptimalen Bedingungen.

Bei herkömmlichen Verfahren werden aufwendige Maßnahmen getroffen, um diese Nachteile zu vermeiden. Es werden sehr hohe Drehzahlen des Rührere angewandt, was einen hohen spezifischen Energieeintrag bedeutet und entsprechende Kühlkapazität erfordert. Auch der Einsatz hoher Volumenanteile nicht mischbarer organischer Flüssigkeiten wurde vorgeschlagen, um eine Emulsion mit geringer Viskosität zu bilden; vgl. beispielsweise DE-A1-3 330 328.2. Hierbei ist jedoch der Verlust an Fermentationsvolumen und der Aufwand für die Abtrennung und Rückgewinnung des Lösungsmittels zu berücksichtigen. Die Installation eines zusätzlichen wandgängigen Rührers oder die Verwendung von Rührern größeren Durchmessers erfordern erhebliche zusätzliche Investitionen und insbesondere einen drastischen Anstieg der Energiekosten. Dies gilt ebenso für den Einsatz statischer Mischer im Rohrreaktor; vgl. beispielsweise EP-A2-0 185 407 = 85.201 863.9.

In Blasensäulen, die sich durch das Fehlen mechanisch bewegter Organe wie Rührer auszeichnen, erfolgt die Vermischung der flüssigen Phase allein durch die Schleppwirkung der aufsteigenden Gasblasen. Bei Vorliegen hochviskoser Medien erwartet der Fachmann geringe Durchmischung und unzureichenden Sauerstoffeintrag. Es ist allerdings bekannt, daß bei Erhöhung des Gasdurchsatzes die Schergeschwindigkeit im Fermenter ansteigt, was die effektive Viskosität der üblicherweise pseudoplastischen Medien nach dem Gesetz von Ostwald - de Waele erniedrigt; vgl. beispielsweise Int. Eng. Chem. Process Des. Dev., 6 (1977) 133-137. Tatsächlich wurde für Reaktoren des Typs Blasensäule schon bei mäßigen Viskositäten eine Verschlechterung des Vermischungsverhaltens mit zunehmender Viskosität beobachtet; vgl. beispielsweise Chem. Eng. J., 8 (1974) 191-197.

Demgegenüber wurde nun erfindungsgemäß überraschenderweise festgestellt, daß Blasensäulen hervorragend geeignet sind zur mikrobiellen Herstellung von exozellulären Polysacchariden, wobei die Fermentationsmedien ausgesprochen hochviskos sind und pseudoplastisches Verhalten aufweisen. Die Eignung von einfachen Blasensäulen ist darauf zurückzuführen, daß es bei hohen Gasdurchsätzen zur Bildung von Großblasen kommt, deren Aufstieg im Reaktor derart für ausreichenden Sauerstoffeintrag und gute Vermischung des Reaktorinhalts sorgt, daß selbst bei hochviskosen Medien keine unbewegten (stagnanten) Zonen in Wandnähe auftreten.

Die Erfindung betrifft demgemäß ein Verfahren zur fermentativen Herstellung exozellulärer Polysaccharide in Blasensäulen, bei dem man durch das Fermentationsmedium die Kultivierungsluft in Reaktoren größeren Durchmessers in Form von Großblasen, in Reaktoen geringeren Durchmessers (< 0,8 m) in Form von Kolbenblasen durchtreten läßt, wobei die Kolbenblasen einen Durchmesser im Bereich von 50 bis 95 % des lichten Säulendurchmessers aufweisen.

Erfindungsgemäß läßt sich also die Durchmischung des Kulturmediums verbessern, ohne daß unbewegte Zonen auftreten. Das gilt auch bei sehr hohen Viskositäten, bei denen sich selbst an der Wandung keine unbewegten Zonen ausbilden. Dadurch kann man sehr hohe Produktkonzentrationen erreichen. Von besonderem Vorteil ist der gegenüber Rührfermentern geringe erforderliche Energieeintrag, der allein aus der Gasexpansion herrührt.

Die zu wählende Gasgeschwindigkeit steigt mit der angestrebten Polysaccharidkonzentration. Anwendbar sind Gasleerrohrgeschwindigkeiten im Bereich von 0,01 bis 1 m/s, vorzugsweise im Bereich von 0,05 bis 0,4 m/s.

Das erfindungsgemäße Verfahren läßt sich mindestens bis zu einem Konsistenzfaktor k des Kulturmediums von 60, vorzugsweise 80 und insbesondere 100 Pa x $s^n$ durchführen. Dasrfindungsgemäße Verfahren kann chargenweise (mit oder ohne Nachfütterung von Nährstoffen) oder kontinuierlich durchgeführt werden. Vorzugsweise arbeitet man in Blasensäulen ohne Einbauten oder Schikanen.

Sollte der Sauerstoffpartialdruck der Kulturbrühe bei einer gewissen Produktkonzentration auf sehr geringe Werte sinken, die eine Minderung der Produktionsgeschwindigkeit bewirken, so kann man die Sauerstoffversorgung beispielsweise dadurch verbessern, daß man den Sauerstoffpartialdruck im zugeführten Gas erhöht (beispielsweise mit einem sauerstoffreicheren Gas als Luft oder durch Druckerhöhung) oder sauerstoffreiche chemische Substanzen (beispielsweise Wasserstoffperoxid) zudosiert.

Nachstehend wird die Erfindung durch Beispiele anhand von Abbildungen näher erläutert.

Es zeigen:

Fig. 1 graphisch die Xanthan-Konzentration (mit Konsistenzfaktor und Fließindex) gegen die Fermentationszeit bei einem Fermentationsbeispiel;

Fig. 2 graphisch die Xanthan-Konzentration gegen die Fermentationszeit bei drei Fermentationsbeispielen; und

Fig. 3 graphisch die Biotrockenmasse (mit Glukose-Konzentration) gegen die Fermentationszeit bei dem Fermentationsbeispiel von Fig. 1.

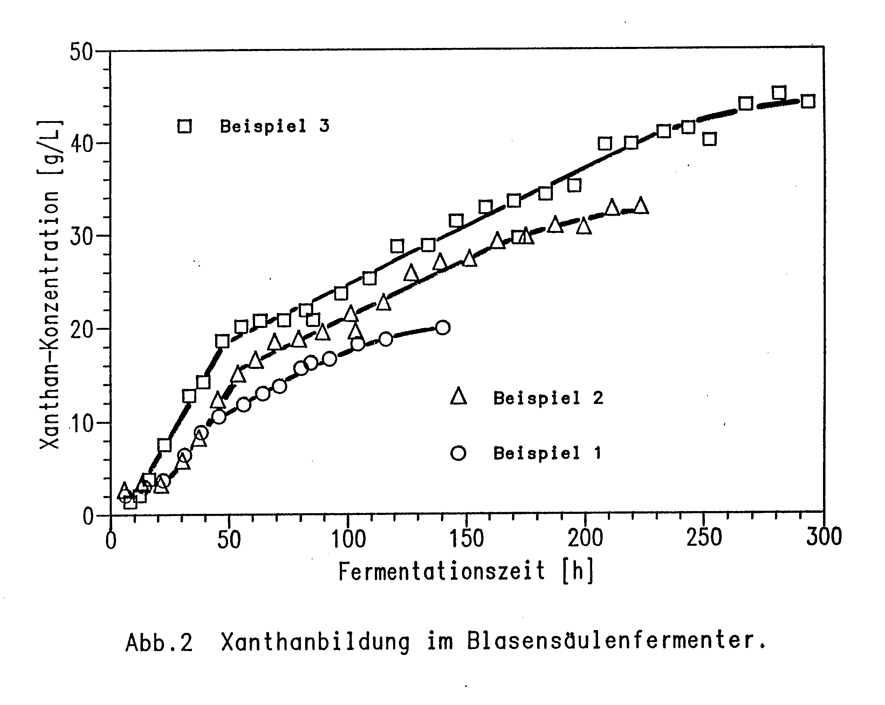

## Beispiel 1: (vgl. Abb. 2)

Xanthomonas campestris B-1459S4L-II wurde in einer Blasensäule von 0,15 m Durchmesser bei einem Füllvolumen von 40 l bei 28 °C gezüchtet. Das Nährmedium der Hauptkultur hatte folgende Zusammensetzung: 50 g/l Glukose, 2,31 g/l Citronensäuremonohydrat, 5 g/l $KH_2PO_4$, 2 g/l $NH_4Cl$, 0,5 g/l $Na_2CO_3$, 0,114 g/l $Na_2SO_4$, 0,163 g/l $MgCl_3$ $6H_2O$, 1,4 mg/l $FeCl_3$, 6,7 mg/l $ZnCl_2$, 29 mg/l $CaCl_2$ $2H_2O$, 6 mg/l $H_3BO_3$. Die sterilisierte Kulturlösung wurde mit 1/9 ihres Volumens einer 24 Stunden alten Vorkultur beimpft. Durch Zudosieren von Natronlauge wurde ein Absinken des pH-Wertes auf unter 7,0 während der Fermentation verhindert. Verdunstungsverluste wurden durch Nachdosieren sterilen Wassers ausgeglichen. Der Fermenter wurde über eine Sinterplatte mit Luft begast; die Gasleerrohrgeschwindigkeit betrug 0,10 m/s (Energieeintrag: 1 kW/m³).

Die Xanthankonzentration betrug nach 50 Stunden Fermentationszeit 10 g/l. Nach 130 Stunden war die Glukose verbraucht und eine Xanthankonzentration von 20 g/l erreicht.

## Beispiel 2: (vgl. Abb. 2)

Wie Beispiel 1, aber eine Gasleerrohrgeschwindigkeit von 0,16 m/s (Energieeintrag: 1,6 kW/m³) und Fed-Batch-Betrieb.

Nach 70 Stunden Fermentationszeit war eine Xanthankonzentration von 18 g/l erreicht. Nach 120 Stunden war die Glukose verbraucht und eine Xanthankonzentration von 22 g/l erzielt. In der Form einer konzentrierten Lösung wurden 33 g/l Glukose zugegeben. Nach 230 h gesamter Fermentationszeit war die Glukose wiederum verbraucht und eine Xanthankonzentration von 33 g/l erzielt.

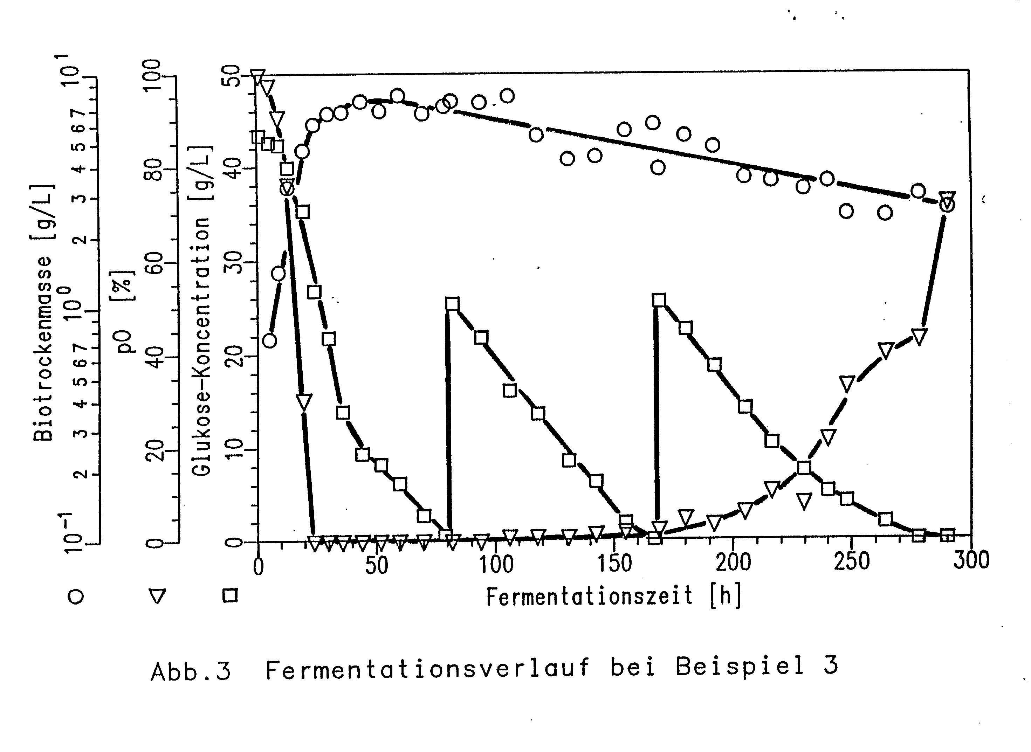

## Beispiel 3: (vgl. Abb. 1, Abb. 2 und Abb. 3)

Wie Beispiel 2, aber ein komplexes Nährmedium für die Hauptkultur mit folgender Zusammensetzung: 50 g/l Glukose, 8 g/l Hefeextrakt, 0,4 g/l Harnstoff, 5 g/l $K_2HPO_4$, 0,2 g/l $MgSO_4$ $7H_2O$.

Nach 55 Stunden betrug die Xanthankonzentration 20 g/l. Nach 80 h war die Glukose verbraucht und eine Xanthankonzentration von 22 g/l erreicht. Nachdosieren auf 25 g/l Glukose führte nach deren Umsetzung bei 160 h gesamter Fermentationszeit auf 33 g/l Xanthan. Nochmaliges Nachdosieren von 25 g/l Glukose ergab nach deren Umsetzung 44 g/l Xanthan bei 270 Stunden gesamter Fermentationszeit.

## Vergleichsbeispiel:

Es wurde eine Vergleichfermentation wie in Beispiel 1, aber in einem 100-l-Rührkessel bei 50 l Füllvolumen durchgeführt. Der Rührkesselfermenter war mit einem zweistufigen Rührer (Durchmesserverhältnis: 0,75) ausgestattet, der mit 300 Umdrehungen/Minute betrieben wurde. Der aus der Differenz der Leistungsaufnahme des Motors im Betriebs- und im Leerzustand ermittelte volumenspezifische Leistungseintrag betrug 2,3 kW/m³. Begast wurde mit Luft bei 0,25 vvm.

Nach 50 Stunden betrug die Xanthankonzentration 8 g/l. Nach 125 h war die Glukose verbraucht und eine Xanthankonzentration von 20 g/l erreicht.

## Ansprüche

1. Verfahren zur fermentativen Herstellung exozellulärer Polysaccharide dadurch **gekennzeichnet,** daß man als Fermentationsreaktoren Blasensäulen oder Turmreaktoren verwendet.

2. Verfahren nach Anspruch 1, dadurch **gekennzeichnet,** daß man bei einem Reaktor eines lichten Säulendurchmessers von > 0.8 m Großblasen und bei einem Reaktor eines lichten Säulendurchmessers von ≦ 0.8 m Kolbenblasen eines Durchmessers im Bereich von 50 bis 90 % des lichten Reaktorsäulendurchmessers durch das Fermentationsmedium treten läßt.

3. Verfahren nach Anspruch 1 oder 2, dadurch **gekennzeichnet,** daß man bei Gasleerrohrgeschwindigkeiten von 0,01 bis 1 m/s, vorzugsweise bei 0,05 bis 0,4 m/s fermentiert.

4. Verfahren nach einem der vorhergehenden Ansprüche, dadurch **gekennzeichnet,** daß man bis zu einem Konsistenzfaktor k des Kulturmediums = 60, vorzugsweise bis = 80 und insbesondere = 100 Pa x $s^n$ oder mehr fermentiert.

5. Verfahren nach einem der vorhergehenden Ansprüche, dadurch **gekennzeichnet,** daß man mit sauerstoffangereicherter Luft, mit Luftdruckerhöhung oder unter Zudosierung von Sauerstoffspendern in das Kulturmedium arbeitet.

6. Verfahren nach einem der vorhergehenden Ansprüche, dadurch **gekennzeichnet,** daß man in Blasensäulen oder Turmreaktoren ohne Einbauten und/oder Schikanen fermentiert.

7. Verfahren nach einem der vorhergehenden Ansprüche, dadurch **gekennzeichnet,** daß man absatzweise (Batch bzw. Repeated Batch), mit wiederholter Substratzufütterung (Fed-Batch) oder kontinuierlich arbeitet.

Abb.1 Xanthan-Konzentration und Fließverhalten (Beispiel 3)

EP 0 363 646 A1

Xanthan-Konzentration [g/L]

Beispiel 3

Beispiel 2

Beispiel 1

△ Beispiel 2

○ Beispiel 1

□ Beispiel 3

Fermentationszeit [h]

Abb.2 Xanthanbildung im Blasensäulenfermenter.

Abb.3   Fermentationsverlauf bei Beispiel 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | BULLETIN DE LA SOCIETE CHIMIQUE DE FRANCE, Nr. 6, 1985, Seiten 1075-1082; C.G. DUSSAP et al.: "Fermenteurs agités sans organes d'agitation mécanique application à la production de polysaccharides exocellulaires" * Insgesamt * | 1-7 | C 12 M 1/04<br>C 12 P 19/04 |
| A | EP-A-0 249 288 (COÖPERATIEVE VERENIGING SUIKER UNIE U.A.) * Insgesamt * | 1-7 | |

RECHERCHIERTE
SACHGEBIETE (Int. Cl.5)

C 12 M
C 12 P

Der vorliegende Recherchenbericht wurde für alle Patentansprüche erstellt

| Recherchenort | Abschlußdatum der Recherche | Prüfer |
|---|---|---|
| DEN HAAG | 05-12-1989 | GROENENDIJK M.S.M. |